# EUROPEAN PATENT SPECIFICATION

(11) **EP 0 916 689 B1**
(45) Date of publication and mention of the grant of the patent: **07.04.2004**
(21) Application number: 98121162.6
(22) Date of filing: 12.11.1998
(51) Int. Cl.: C08G 77/46, C08G 77/38, C08G 77/02, A61K 7/06

(54) **Silicone terpolymers with high refractive indices**
Silikon-Terpolymere mit hohem Brechungsindex
Terpolymères de silicone à indice de réfraction élevé

(30) Priority: 12.11.1997 US 65211 P
(43) Date of publication of application: 19.05.1999
(73) Proprietor: CROMPTON CORPORATION, Middlebury, Connecticut 06749 (US)
(72) Inventor: Czech, Anna, Cortlandt Manor, NY 10566 (US); Cooke, Jeffrey A., Brewster, NY 10509 (US)
(74) Representative: Wibbelmann, Jobst, Dr., Dipl.-Chem.

(56) References cited:
- EP-A- 0 779 319
- WO-A-93/19074
- WO-A-94/08557
- GB-A- 2 297 757
- US-A- 5 334 227
- US-A- 5 542 960
- US-A- 5 565 194

## Description

### Field of the Invention

The present invention concerns linear substituted polysiloxanes having high refractive indices.

### Background

Phenyl and phenethyl groups pendant polysiloxanes with high refractive indices are useful in applications that benefit from improved surface slickness and gloss, such as hair care and coatings. See, e.g., U.S. Patent No. 3,389,159 and German Patent 56,102 which provide synthesis of polysiloxanes with phenyl substitution. A review article by M. Berthiaume et al., J. Soc. Cosmetic Chemists, No. 1, 1997, provides performance data of the phenyl-substituted and phenethyl-substituted silicones on hair.

WO 94/08557 provides examples of the hair care compositions providing conditioning and enhanced shine to the hair comprising the combination of high refractive index, nonvolatile aryl substituted polysiloxanes, a novolatile spreading agent which is compatible with the polysiloxane fluid and a carrier suitable for hair care application. However, generally, phenyl substituted polysiloxanes are not readily compatible with personal care matrices.

US Patent Nos. 5,334,227 and 5,542,960 teach a class of silicone polymers modified with both phenol derivatives substituents, such as eugenol, and polyether grafted onto a silicone backbone for diesel antifoam applications. Such polysiloxanes tend to have viscosities which are too high for use in personal care formulations though.

EP 0 779 319 discloses silicone foam control agents for hydrocarbon liquids comprising organopolysiloxane compounds bearing a polyalkyleneoxide and unsubstituted phenyl-alkyl or phenyl-O-alkyl groups.

US 5,565,194 describes terpolymers with alkyl/aryl and polyether functional groups wherein the polymer groups are combinations of two different polymers.

WO 93/19074 teaches a class of siloxane compounds bearing polyoxybutyl side chains and alkyl/aryl groups.

GB 2,297,757 discloses low viscosity organofunctionalized siloxysilicates with branched/cross-linked silicon structures.

### Brief Description of the Drawings

Figure 1 is Gloss of Hair Samples according to the examples.
Figure 2 is the Gloss of Terpolymers in UV cured acrylate formulations.
Figure 3 is a correlation between gloss and the refractive index of the terpolymer used.

### Summary of the Invention

The present invention relates to fluids comprising linear terpolymers of (a) aryl component including phenyl or substituted phenyl groups with at least one hydrocarbon side chain of 2-5 carbons; (b) polyalkyleneoxide, and (c) silicone. These terpolymers have refractive indices (RI) > 1.47. Fluids of the present invention when incorporated in compositions employed as surface treatments improve slip and gloss of the substrates to which the compositions are applied. They also offer an advantage over phenyl-substituted and phenethyl-substituted materials in compatibility with media commonly used in hair care applications.

### Detailed Description of the Invention

The present invention is directed more specifically to polysiloxanes of the general formula (I)

[O_{1/2}MeSi(Q)O_{1/2}]_{f}[O_{1/2}SiMe₂Q]_{g}

wherein:
Q is R, R¹ or R²; but at least one Q is R¹ and at least one Q is R².
R is a monovalent saturated straight, branched, or cyclic alkyl group having 1 to 10 carbon atoms. Each of the R groups present may be the same or different from one another. Examples of R groups include methyl, ethyl, butyl, hexyl. Of these, lower alkyl groups (C₁-C₄) are preferred. Most preferably R is methyl.
R¹ is -R³-C₆H₃R⁴R⁵ where R³ is a divalent hydrocarbon group of 2-5 carbons, R⁴ and R⁵ are the same or different and selected from a C₁-C₆ alkyl, a C₁-C₆ alkoxy group, a tri-(C₁-C₆-alkyl)siloxy group, or an acyloxy group R^{A}C(O)O- (wherein R^{A} is C₁-C₁₀ alkyl, aryl (preferably phenyl), or an aryl-C₁-C₁₀-alkyl (such as benzyl or phenethyl)), an amide, RC(O)R'CO₂H (wherein R' is a C₁-C₁₀ divalent hydrocarbon), (-OCᵥH₂ᵥ)ₗOR⁷ (wherein v is 2 to 4, 1 is 1 to 10 and R⁷ is hydrogen or R above); preferably R⁴ and R⁵ are in the para and meta positions on the phenyl ring; alkoxy groups are the most preferred. Preferably, at least one of R⁴ and R⁵ is an alkoxy group.
R² is represented by formula -(CₘH₂ₘ)(OCₙH₂ₙ)ₚOR⁶ (II), where R⁶ can be hydrogen, R or acetyl. "m" is an integer greater than zero, each n is 2 to 4, p is from 0 to 100. Preferably, "m" ranges from 2 to 8, and n is 2 or 3, most preferably 2. Mixtures of different n are possible within one R² group.

In the linear siloxanes, g is 2 and f is from 1 to 500. Preferably, f is from 5 to 100, most preferably 10 to 50.

The actual siloxane chain length does not affect the refraction index; rather it is the ratio of the number of R¹ to R and R¹ to R², with R¹ increasing RI and R and R² decreasing the RI. The ratio of the number R¹ to R + R² is 1:5 to 20:1, more preferably 1:2 to 10:1. As stated, this ratio is modified to achieve an RI > 1.47 so that the polysiloxane will have an appropriate gloss level.

Unlike terpolymers defined by US patent No. 5,334,227, terpolymers of the present invention do not contain phenolic functionality; presence of free hydroxyl groups on aromatic substituents resulted in high viscosity copolymers with less desirable tactile properties.

The index of refraction is the ratio of the speed of light in a vacuum to the speed of light in the medium. This is a dimensionless parameter which ranges between 1.3 and 1.55 for most of organic liquids. The refractive index is measured using a beam of monochromatic light, typically, the yellow light of the sodium D line (wavelength λ = 589.3 nm). Thus n²⁵_{D} indicates the wavelength used D, and the temperature 25°C. Other wavelengths used are C and F line of hydrogen and G line of mercury, but for purposes of the present invention references are to the D line.

### Manufacture

Terpolymers of the present invention can be prepared by platinum catalyzed hydrosilation reaction of an allyl started phenyl derivative (e.g., of formula CH₂=CHCH₂C₆H₃(OCH₃)(OR₃) ) and the terminally unsaturated polyalkyleneoxide (of formula CH₂=CH-(Cₘ₋₂H₂ₘ₋₄)(OCₙH₂ₙ)ₚOR⁶ with hydrogen-substituted silanic fluid under typical hydrosilation conditions, such as those disclosed in U.S. Patent Nos. 3,299,112 and 5,334,227, which are incorporated herein by reference.

According to the Comprehensive Handbook on Hydrosilation, edited by Bogdan Marciniec ( Pergamon Press, First Edition 1992) (incorporated by reference herein), hydrosilation is a term that describes addition reaction of silicon hydrides to the multiple bonds such as carbon-carbon, carbon-oxygen, carbon-nitrogen and nitrogen-oxygen. The reaction can proceed according to a free-radical mechanism or when catalyzed by transition metal salts and complexes as well as other catalysts the mechanism is predominantly polar. The catalyst can be selected from the group of platinum, palladium, rhodium and ruthenium less commonly nickel or aluminum in the form suitable for either homo- or heterogenous catalysis. Most commonly hydrosilation reactions are catalyzed by platinum catalyst such as chloroplatinic acid.

Eugenol and some of its ethers and esters of the foregoing formula (I) are commercially available. Eugenol ethers and esters of formula (I) can be prepared according to known procedures suitable for preparation of aromatic ethers (e.g., C. Moreau, F. Roessac, J.M. Conia, Tetrahedron Lett., 3527, 1970; H.N. Grant, V. Prelog, R.P.A. Sneeden, Helv. Chim. Acta, **46,** 415, 1963) and of aromatic esters (e.g., V.O. Illi, Tetrahedron Lett., 2431, 1979; P.A. Stadler, Helv. Chim. Acta, **61,** 1675, 1978), which are incorporated herein by reference.

### Use

The terpolymers of the present invention can be used neat, or for ease of application they can be applied dissolved, dispersed or emulsified in a suitable liquid medium, such as water, alcohol, glycols, other silicones, etc. Particular silicones which may be used are cyclic siloxanes and low viscosity oils (< 100 cps).

Standard surfactants may be used in such liquids, such as anionic, nonionic and cationic surfactants. Particular classes of surfactant would be polyether modified polysiloxanes and polysorbates.

Other additives typically employed in hair care applications can be included with the terpolymers of the present invention or applied separately to the substrate. Such additives can include resins, preservatives, biocides, biostats, pigments, dyes, fragrances, pH buffers, antifoams and defoamers.

Personal care applications, especially those where clarity may be an issue may be made with terpolymers of the present invention, including nail polishes, anti-persperants, body washes, mascara, soaps, detergents, etc. Examples of the hair care formulations that can benefit from the incorporations of the terpolymers of the present invention include shampoos, rinse conditioners, hair dressings, leave-in conditioners, styling mousses and hair spray. See, e.g., WO 94/08857, which is incorporated herein by reference.

Moreover, terpolymers of the present invention may be used in other applications, more preferably coating compositions, such as car care, decorative coatings and overprint varnishes wherein the gloss of the final product is important.

### Examples

The following specific examples are set forth for illustration only and are not to be construed as limiting the present invention. For the examples M is (Me)₃SiO_{1/2}; D is [O_{1/2}Me₂SiO_{1/2}]; D* is [O_{1/2}MeSiR¹O_{1/2}]; and D' is [O_{1/2}MeSiR²O_{1/2}]. The instrument used to determine refractive indices of the polysiloxanes of the present invention was LEICA ABBE MARK II Refractometer, utilizing line D light and the measurements were taken at 25°C.

### Example 1

Hair evaluations were performed on 10 inch (25.4 cm) tresses of Oriental hair. Hair tresses were soaked in toluene for 10 minutes to remove any natural oils that could contribute to the gloss. Terpolymers I-III (Table 1) were dissolved in isopropanol to a concentration of 0.1wt. % , and 1cc of each solution was applied and worked through the tresses; control tresses were treated with isopropanol. All tresses were then air dried for 1 hour. Hair evaluations were performed by a panel of three observers who compared gloss and feel of the treated samples to the untreated control. The results are summarized in Table 2 .

**Table 1.**

| Structural Information | | | |
|---|---|---|---|
| | Formula | Refractive Index | Viscosity (cps) - 25°C |
| Terpolymer I (control derived from eugenol) | MD₅D*₆D"₁M | 1.50 | 3700 |
| | wherein R¹ is -C₃H₆-C₆H₃(OH)(OCH₃) | | |
| | wherein R² is -(CH₂)₃(OC₂H₄)_{b}OCH₃, with b a number such that R has an average MW=350 | | |
| Terpolymer II | MD₅D*₆D"₁M | 1.49 | 880 |
| | wherein R¹ -C₃H₆-C₆H₃(OCH₃)₂ | | |
| | R² is -(CH₂)₃(OC₂H₄)_{b}OCH₃ with R² of average MW = 350 | | |
| Terpolymer III | MD₅D*_{6.5}D"_{0.5}M | 1.51 | 942 |
| | wherein R¹ -C₃H₆-C₆H₃(OCH₃)₂ | | |
| | R² is (CH₂)₃(OC₂H₄)_{b}OCH₃ with R² of average MW=350 | | |

**Table 2.**

| Hair Testing Results | | |
|---|---|---|
| Hair Sample | Gloss Compared to Control | After-Feel Compared to Control |
| Terpolymer I | improved | softer, tacky |
| Terpolymer II | improved | smoother, softer |
| Terpolymer III | improved | smoother, softer |

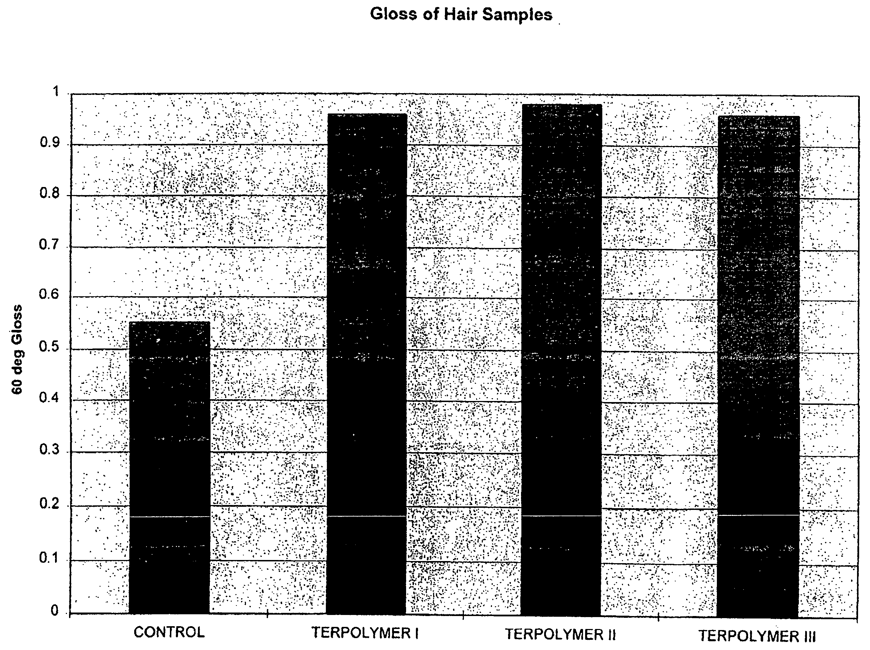

All terpolymers imparted gloss to hair. The terpolymer structures that had been modified with eugenol ether offered better after-feel. In addition, gloss of these hair tresses has been assessed using the following non-subjective test method: 60º Gloss - Gloss data were generated using a Gardner Micro-Tri-Gloss Meter. Two tresses were prepared and measured for each finish. Each tress was subjected to eight readings taken at 45° increments by rotating the meter on the hair tress. An average of the eight readings was calculated for each hair tress. A data point was generated from the average of two tresses for each finish. The data is presented in Figure 1. Gloss values of hair samples treated with high refractive index copolymers were higher than the untreated control.

### Example 2

The 20° gloss of Terpolymers A-D (as given in Table 3) was evaluated in the following model acrylate UV cured overprint varnish formulation:

| *Component* | *Manuf.* | *pph* |
|---|---|---|
| EBECRYL 745 | UCB Radcure | 35 |
| Trimethylolpropane triacrylate | Aldrich Chem. Co. | 47 |
| Tripropylene glycol diacrylate | Aldrich Chem. Co. | 10 |
| IRGACURE 184 | Ciba Specialty Chem. Co. | 7 |
| Terpolymer | | 1 |

The formulations were drawn down on Leneta unlacquered opacity charts using a TMI K-Control coater equipped with a #3 wire wound rod (5 panels per group for a total of 20 panels). The drawdowns were completed at #4 speed, and the panels cured at 300 mJ/cm² using a bench top UV curing unit (UV Process Supply Inc.). The 20° gloss of the cured panels was measured one hour after curing using a Mini Tri-Gloss Portable Glossmeter (Byk-Gardner Inc.).

**Table 3**

| Structural Information for Terpolymers A, B, C, D. | | |
|---|---|---|
| | Formula | Refractive Index |
| Terpolymer A | MD ₅ D* ₃ D'' ₄ M | 1.4723 |
| | wherein R ¹ is -C ₃ H ₆ -C ₆ H ₃ (OCH ₃ ) ₂ | |
| | R ² is (CH ₂ ) ₃ (OC ₂ H ₄ ) _{b} OCH ₃ with R ² of average MW=350 | |
| Terpolymer B | MD ₅ D* ₆ D'' ₁ M | 1.4949 |
| | wherein R ¹ -C ₃ H ₆ -C ₆ H ₃ (OCH ₃ ) ₂ | |
| | R ² is (CH ₂ ) ₃ (OC ₂ H ₄ ) _{b} OCH ₃ with R ² of average MW=350 | |
| Terpolymer C | MD ₅ D* ₆ D'' ₁ M | 1.4950 |
| | wherein R ¹ -C ₃ H ₆ -C ₆ H ₃ (OCH ₃ ) ₂ | |
| | R ² is (CH ₂ ) ₃ (OC ₂ H ₄ ) _{b} OCH ₃ with R ² of average MW=350 | |
| Terpolymer D | MD ₅ D* _{6.5} D'' _{0.5} M | 1.5150 |
| | wherein R ¹ is -C ₃ H ₆ -C ₆ H ₃ (OCH ₃ ) ₂ | |
| | wherein R ² is (CH ₂ ) ₃ (OC ₂ H ₄ ) _{b} OCH ₃ with R ² of average MW=350 | |

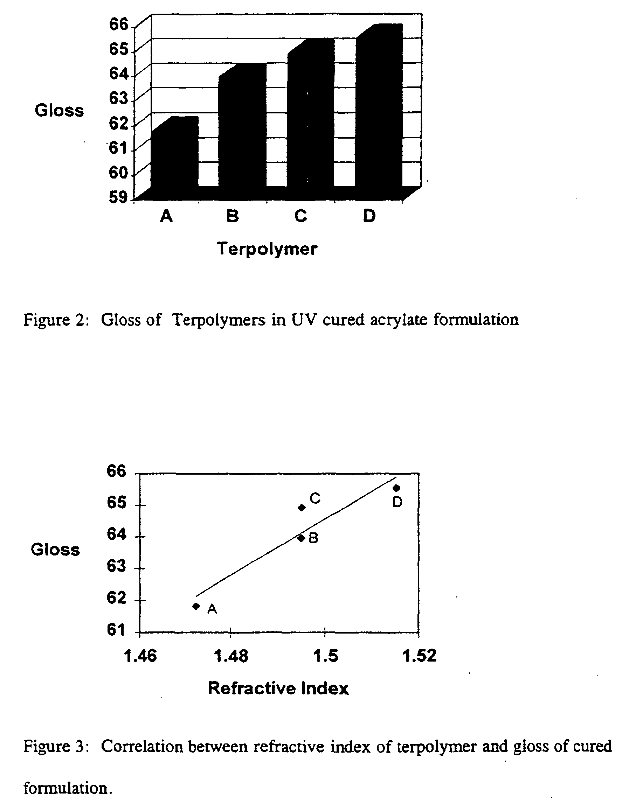

The results are summarized in Figures 2 and 3. Figure 2 shows the 20° gloss measurements of the four terpolymers, while Figure 3 shows the correlation between the gloss of the formulation and the refractive index of the terpolymer. Clearly as the refractive index of the terpolymer increases, the measured gloss of the cured formulation increases.

### Example 3 - Evaluation of Gloss Enhancment in the Tire Dressing

"Son of a Gun", a commercially available tire dressing formulation, as received and containing 0.2% of the Terpolymer II were applied onto SBR (styrene-butadiene rubber) sheets rinsed with water to remove excess dust. 0.2g of the formulation was applied in 3-4 draw-downs with the manual draw-down machine, wire#3. The 60° gloss of the air dried samples was measured using a Mini Tri-Gloss Portable Glossmeter (Byk-Gardner Inc.). Results are summarized in Table 4.

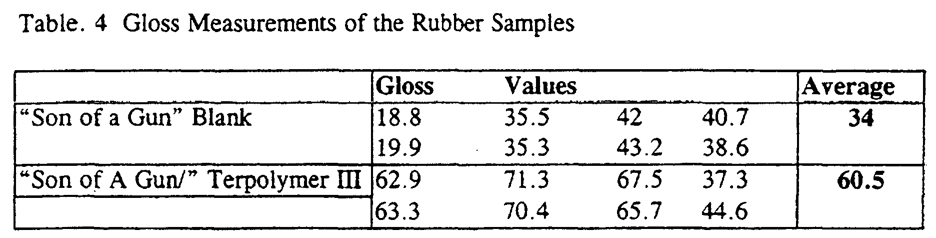

Incorporation of the Terpolymer II into the tire dressing formulation enhanced the gloss of the treated rubber.

### Example 4 - Formulations

The following are examples of the model hair care formulations featuring polysiloxanes of the present invention:

### Gloss Enhancing Clear Shampoo

### Formula:

| **Ingredients** | **Wt Percent** |
|---|---|
| WITCOLATE NH (Witco Corp.)(Ammonium Lauryl Sulfate, 28%) | 35.7 |
| WITCAMIDE CMEA (Witco Corp.) (Cocamide MEA) | 2.2 |
| PEG-120 Methyl Glucose Dioleate, | 2.0 |
| Terpolymer III | 0.3 |
| Citric Acid, anhydrous | 0.4 |
| REWOTERIC AMB14 (Witco Corp.) (Coacamidopropyl Betaine, 35%) | 10.0 |
| Deionized Water | qs |
| Preservative | qs |

*Mixing Instructions:* With propeller agitation, mix deionized water and ammonium lauryl sulfate. Heat to 45°C and add remaining ingredients in the order listed, waiting for each ingredient to dissolve before adding the next. Cool to room temperature. At the recommended use level, Terpolymer III may cause slight haze and up to 20 % viscosity decrease depending on the primary surfactants system. Clarity of the shampoo may be improved by pre-blending Terpolymer III with dipropylene glycol at 1:5 ratio.

### Gloss Enhancing Hair Conditioner

### Formula:

| **Ingredients** | **Wt Percent** |
|---|---|
| Terpolymer III | 0.30 |
| VARISOFT CRC (Witco Corp.) (quatemulsifier) | 5.00 |
| "ACTISEA" TM 100 (Active Organics, Inc.) (seaweed extract) | 0.50 |
| DL "PANTHENOL" (Tri-K Industries, Inc.) (provitamin B) | 0.20 |
| Citric Acid | 0.05 |
| Deionized Water | qs |
| Preservative | qs |

*Mixing Instructions:* While agitating the water, add citric acid and Varisoft® CRC. With mixing, heat to 75-80°C and add remaining ingredients. Cool to room temperature with mixing. Adjust to pH 4.5-5.5 with citric acid if necessary. Add preservative as needed.

### Low VOC Pump Hair Spray with Enhanced Gloss

### Formula:

| **Ingredients** | **Wt Percent** |
|---|---|
| BALANCE™, 50% Resin-National Starch (polyacrylate) | 8.0 |
| Terpolymer III | 0.1 |
| Ethanol, denatured | 55.0 |
| Aminomethyl Propanol (MPA) to pH 7 | qs |
| Deionized Water | qs |

*Mixing Instructions:* Combine Balance Resin, water ethanol and Terpolymer III. While agitating, add MPA.

### Clear Styling Gel with Enhanced Gloss

### Formula:

| **Ingredients** | **Wt Percent** |
|---|---|
| Phase A | |
| Deionized Water | 67.0 |
| Carbomer 940 -BF Goodrich - polyacrylic acid | 0.8 |

| Phase B | |
|---|---|
| Triethanolamine | 0.8 |

| Phase C | |
|---|---|
| Kemester USP (Witco Corp.) (Glycerin) | 3.0 |
| Propylene Glycol | 3.0 |

| Phase D | |
|---|---|
| Deionized Water | 22.3 |
| PVP (PVP-K30) (ISP) (polyvinyl pyrrolidone) | 2.0 |

| Phase E | |
|---|---|
| Terpolymer III | 0.1 |
| TWEEN 20 (ICI) (polysorbate) | 1.0 |

*Mixing Instructions:* Combine ingredients of Phase A, add Phase B with mixing. Add phase C. Premix ingredients of Phase D and add to the batch while mixing. Add premixed components of Phase E and mix until uniform.

## Claims

1. Linear polysiloxanes exhibiting refractive indices n²⁵_{D} > 1.47 of the general formula (I)
[O_{½}MeSi(Q)O_{½}]_{f}[O_{½}SiMe₂Q]_{g}
wherein:
Q is R, R¹ or R²; but at least one Q is R¹ and at least one Q is R².
R is a monovalent saturated straight, branched, or cyclic alkyl group having 1 to 10 carbon atoms, wherein each of the R groups present may be the same or different from one another.
R¹ is -R³-C₆H₃R⁴R⁵ where R³ is a divalent hydrocarbon group of 2-5 carbons, R⁴ and R⁵ are the same or different and selected from a C₁-C₆ alkyl, a C₁-C₆ alkoxy group, a tri-(C₁-C₆-alkyl)siloxy group, or an acyloxy group R^{A}C(O)O- (wherein R^{A} is C₁ -C₁₀ alkyl, aryl , or an aryl-C₁ -C₁₀-alkyl),
an amide,RC(O)R'CO₂H (wherein R' is a C₁-C₁₀ divalent hydrocarbon), (-OCᵥH₂ᵥ)₁OR⁷ (wherein v is 2 to 4, 1 is 1 to 10 and R⁷ is hydrogen or R above);
R² is represented by formula -(CₘH₂ₘ)(OCₙH₂ₙ)ₚOR⁶ (II), where R⁶ can be hydrogen, R or acetyl; "m" is an integer greater than zero, each n is 2 to 4, p is from 0 to 100;
g is 2 and f is 1 to 500.

2. A polysiloxane according to claim 1 wherein R is methyl.

3. A polysiloxane according to claim 2 wherein R⁴ and R⁵ are in the para and meta positions on the phenyl ring.

4. A polysiloxane according to claim 3 wherein at least one of R⁴ and R⁵ are alkoxy groups.

5. A polysiloxane according to claim 1 wherein R^{A} is phenyl, benzyl or phenethyl.

6. A composition comprising a polysiloxane according to claim 1 and an emulsifier.

7. A coating composition incorporating the polysiloxane of claim 2.

8. A hair care formulation incorporating the polysiloxane of claim 2.

## Patentansprüche

1. Lineare Polysiloxane, die einen Brechungsindex n²⁵_{D} > 1,47 aufweisen, der allgemeinen Formel (I)
[O_{1/2}MeSi(Q)O_{1/2}]_{f}[O_{1/2}SiMe₂Q]_{g}
in der
Q für R, R¹ oder R² steht; aber mindestens ein Q für R¹ steht und mindestens ein Q für R² steht;
R eine einwertige gesättigte gerade, verzweigte oder cyclische Alkylgruppe mit 1 bis 10 Kohlenstoffatomen ist, wobei jede der vorhandenen Gruppen R gleich oder verschieden voneinander sein kann;
R¹ für -R³-C₆H₃R⁴R⁵ steht, worin R³ eine zweiwertige Kohlenwasserstoffgruppe mit 2 - 5 Kohlenstoffen ist, R⁴ und R⁵ gleich oder verschieden sind und aus einer (C₁-C₆)-Alkyl-, einer (C₁-C₆)-Alkoxygruppe, einer Tri-((C₁-C₆)-alkyl)siloxygruppe oder einer Acyloxygruppe R^{A}C(O)O- (worin R^{A} für (C₁-C₁₀)-Alkyl, Aryl oder ein Aryl-(C₁-C₁₀)-alkyl steht), einem Amid, RC(O)R'CO₂H (worin R' ein zweiwertiger (C₁-C₁₀)-Kohlenwasserstoff ist), (-OCᵥH₂ᵥ)ₗOR⁷ (worin v für 2 bis 4 steht, I für 1 bis 10 steht und R⁷ Wasserstoff oder R oben ist) ausgewählt sind;
R² durch die Formel -(CₘH₂ₘ)(OCₙH₂ₙ)ₚOR⁶ (II) dargestellt wird, worin R⁶ Wasserstoff, R oder Acetyl sein kann; "m" eine ganze Zahl größer null ist, jedes n für 2 bis 4 steht, p für 0 bis 100 steht; g für 2 steht und f für 1 bis 500 steht.

2. Polysiloxan nach Anspruch 1, in dem R Methyl ist.

3. Polysiloxan nach Anspruch 2, in dem R⁴ und R⁵ in den para- und meta-Positionen am Phenylring vorliegen.

4. Polysiloxan nach Anspruch 3, in dem mindestens eines von R⁴ und R⁵ eine Alkoxygruppe ist.

5. Polysiloxan nach Anspruch 1, in dem R^{A} Phenyl, Benzyl oder Phenethyl ist.

6. Zusammensetzung, umfassend ein Polysiloxan nach Anspruch 1 und einen Emulgator.

7. Beschichtungsmittelzusammensetzung, welche das Polysiloxan nach Anspruch 2 enthält.

8. Haarpflegeformulierung, welche das Polysiloxan nach Anspruch 2 enthält.

## Revendications

1. Polysiloxanes linéaires présentant des indices de réfraction n²⁵_{D} > 1,47 répondant à la formule générale (1)
[O_{1/2}MeSi(Q)O_{1/2}]_{f}[O_{1/2}SiMe₂Q]_{g}
dans laquelle
Q est R, R¹ ou R² ; mais au moins un Q est R¹ et au moins un Q est R².
R est un groupe alkyle monovalent, saturé, rectiligne, ramifié ou cyclique ayant de 1 à 10 atomes de carbone, dans lequel chacun des groupes présents peut être identique ou différent.
R¹ est -R³-C₆H₃R⁴R⁵ où R³ est un groupe hydrocarbure divalent de 2 à 5 atomes de carbone, R⁴ et R⁵ sont identiques ou différents et choisis parmi un alkyle en C₁-C₆, un groupe alkoxy en C₁-C₆, un groupe tri-(alkyle en C₁-C₆)siloxy ou un groupe acyloxy R^{A}C(O)O- (dans lequel R^{A} est aryle en C₁ -C₁₀, aryle ou un alkyle aryle en C₁-C₁₀),
un amide, RC(O)R'CO₂H (où R' est un hydrocarbure divalent en C₁-C₁₀), (-OCᵥH₂ᵥ)ₗOR⁷ (où v est 2 à 4, I est compris de 1 à 10 et R⁷ est l'hydrogène ou R ci-dessus) ;
R² est représenté par la formule -(CₘH₂ₘ)(OCₙH₂ₙ)ₚOR⁶ (II), dans laquelle R⁶ peut être l'hydrogène, R ou l'acétyle ; "m" est un nombre entier supérieur à zéro, chaque n est compris de 2 à 4, p est compris de 0 à 100 ;
g est 2 et f est compris de 1 à 500.

2. Polysiloxane selon la revendication 1, dans lequel R est le méthyle.

3. Polysiloxane selon la revendication 2, dans lequel R⁴ et R⁵ sont dans les positions para et méta sur le cycle phényle.

4. Polysiloxane selon la revendication 3, dans lequel au moins un de R⁴ et de R⁵ sont des groupes alkoxy.

5. Polysiloxane selon la revendication 1, dans lequel R^{A} est le phényle, le benzyle ou le phénéthyle.

6. Composition comprenant un polysiloxane selon la revendication 1 et un émulsifiant.

7. Composition d'enrobage comprenant le polysiloxane selon la revendication 2.

8. Formulation pour soins capillaires comprenant le polysiloxane de la revendication 2.
